# EUROPEAN PATENT APPLICATION

(11) **EP 3 015 062 A1**
(43) Date of publication of application: **04.05.2016**
(21) Application number: 14191122.2
(22) Date of filing: 30.10.2014
(51) Int. Cl.: A61B 5/024, A61B 5/00

(54) **Optical sensor arrangement for an optical measurement of biological parameters and watch comprising the optical sensor arrangement**

(71) Applicant: ams AG, 8141 Unterpremstätten (AT)
(72) Inventor: Trattler, Peter, 8055 Graz (AT); Meitz, Michael, 8041 Graz (AT)
(74) Representative: Epping - Hermann - Fischer

(57) **Abstract**

Optical sensor arrangement for an optical measurement of biological parameters, in particular for an optical heart rate measurement comprises a light emitting device and a light detector. A substrate comprises a first main surface onto which the light emitting device and the light detector are mounted. A package encloses the light emitting device, the light detector and the substrate and comprises a second main surface facing the first main surface. Furthermore, the package comprises an optical light barrier designed to block light to be emitted by the light emitting device from directly reaching the light detector. A light guiding element connected to the second main surface and designed for coupling light to be emitted by the light emitting device into and/or out of a human skin tissue. Finally, a watch, in particular a wrist watch, comprises the optical sensor arrangement.

## Description

This invention relates to an optical sensor arrangement for an optical measurement of biological parameters, in particular for an optical heart rate measurement. Furthermore, the invention relates to a watch, in particular to a wrist watch, comprising the optical sensor arrangement.

In recent years, mobile devices like Smartphones have been equipped with an increasing amount of sensors including magnetic sensors, GPS (Global Positioning System) sensors, and inclination sensors. Optical sensors in mobile devices, for example, are used for proximity, ambient light and color detection. This development has triggered an increasing demand for applications to monitor and collect health and fitness data from the various sensors. There have been first attempts to use sensors for optical measurement of biological parameters. These attempts involve the use of optical sensors in a wrist watch which can be closely attached to the wrist of a person.

Photoplesmythography (PPG) employs light travelling through biological tissue and allows an optical measurement of the pulsatile wave and therefore heart rate. Briefly, PPG is based on the following principle. Light emitted from a light emitting device is coupled into the human skin. As the blood pressure changes with each heartbeat, the diameter of the blood vessels changes as well. The light penetrating into the skin is slightly modulated by the physical movement of blood vessels. Eventually, the light is reflected or back-scattered from the skin tissue and can be collected by an optical sensor. The corresponding signal can be demodulated in the sensor electronics and provides a measure of the change of blood pressure and, thus, the heartbeat.

Typically, however, the sensor signal is rather weak and superimposed with motion artefacts and pressure disturbances. This makes it difficult to demodulate the sensor signal in order to come up with an accurate heart rate measurement.

Thus, it is an objective of the present invention to provide an optical sensor arrangement for an optical measurement of biological parameters and a watch comprising the optical sensor arrangement which improves on the wear ability and accuracy of optical measurement of biological parameters.

This objective is solved by the subject matter of the independent claims. Further developments and embodiments relate to dependent claims.

According to an aspect of the invention, an optical sensor arrangement for an optical measurement of biological parameters, in particular for an optical heart rate measurement, comprises a light emitting device and a light detector which are mounted to a substrate. The substrate comprises a first main surface onto which the light emitting device and the light detector are mounted.

The optical sensor arrangement further comprises a package enclosing the light emitting device, the light detector and the substrate. The package has an optical light barrier which is designed to block light to be emitted by the light emitting device from directly reaching the light detector.

Furthermore, the package comprises a second main surface facing the first main surface. A light guiding element is connected to the first and/or second main surface. The light guiding element is designed for coupling light to be emitted by the light emitting device into and/or coupling light out of a human skin tissue.

The general operation and measurement principle will be discussed in the following.

The light emitting device of the optical sensor arrangement is used to emit light into a biological probe, for example, human skin tissue. The optical barrier prevents light from directly reaching the light detector without penetrating through the biological probe. This assures that most light will be coupled into the tissue and travels along the tissue layers. The amount of light entering the biological probe depends on various parameters, for example, on its wavelength and the corresponding depth of penetration. Eventually, part of the light will be reflected or backscattered from the tissue. The reflected or backscattered light can be detected by means of the light detector. The amount of detected light is a measure of the biological parameter in question.

One possible application is an optical measurement of heart rates. Preferably, the light emitted from the light emitting device is coupled into the wrist area of a person, for example, by incorporating the optical sensor arrangement into a smart device like a wrist watch or a smart band.

The important skin layer for PPG optical heart rate measurement is the dermis. The epidermis on the contrary does not have any blood vessels, and therefore no pulsatile blood. Thus, light reflected and backscattered from the epidermis does not provide any information on blood pressure whatsoever. When traveling through the dermis the blood pressure will undergo changes and the diameter of the blood vessels will change according to the heart beat. This physical movement will affect how the light travels through the dermis layer and its intensity will be modulated. In turn, this modulation includes clues on the heartbeat. Eventually, light will be scattered within the dermis layer and reaches the light detector. This way the light detector can detect the modulation and generate a sensor signal which can then be demodulated in order to derive the heartbeat.

The optical measurement of biological parameters and, in particular, the optical heart rate measurement is supported by the light guiding element. The optical sensor arrangement can be placed on the biological probe, e.g. human skin tissue, such that the probe is in close contact to the light guiding element. Depending on the position of the light guiding element on the second surface the light guiding element either couples light into the human skin tissue or collects light reflected or backscattered from the tissue to be detected by the light detector.

The light guiding element does not necessarily have to be an optical lens or structure of high optical grade. For example, an optical lens would have a defined focal point. It is sufficient, however, to have light guided into a particular skin layer without the need of a defined focus in a certain penetration depth. Similarly, when light is collected after being reflected or back scattered is not necessary to focus the light on a particular point on the light detector for as long as a sufficient amount of light reaches the light detector's sensitive area.

Based on the aforementioned, properties of the light guiding element can be defined as an element which is able to guide light by means of refraction and reflection, i.e. change the direction of traveling towards a desired position.

Furthermore, the light guiding element can alternatively be used for coupling light into or out of a human skin tissue. However, it is also possible to use more than a single light guiding element. For example, one element is used for coupling light into and another light guiding element for coupling light out of a human skin tissue. In the following, the features discussed for a single light guiding element can be applied to the case with more than a single light guiding element present in the optical sensor arrangement. The light guiding element may comprise more than a single element, like an array or rods in a circular or oval pattern.

The light guiding element can be fabricated by using spray casting at wafer level or by means of wafer level optics. The substrate may comprise a printed circuit board (PCB) or a lead frame to establish electronic connectivity within the optical sensor arrangement and/or to external components. Preferably, the optical sensor arrangement is integrated into an integrated circuit. The optical sensor arrangement may comprise more than a single light emitting device. For example, the light emitting device may comprise one or more light emitting diodes. The light detector may also comprise several individual elements, like photodiodes.

The optical sensor arrangement has several advantages. For one, the package constitutes a mechanical fixture to keep the sensor and its components in place, for example, when the optical sensor arrangement is in contact to human skin. In addition, the light guiding element facilitates channeling light into in the skin, e.g. the dermis layer and/or collecting light from the skin for detection. This way, it is also possible to avoid any air gap which would lead to refraction and loss of light intensity at the air-to-light guiding element interface. Thus, the light guiding element improves signal quality by providing a better signal-to-noise ratio. At the same time the light guiding element reduces power consumption of the light emitting device while maintaining signal quality.

Typically, an optical sensor arrangement of the kind presented above can be used in a mobile device like a wrist watch. In such an environment the light guiding element keeps the sensor mechanically fixed on the skin of the person using the watch. This reduces motion artefacts and pressure disturbances. Therefore the optical sensor arrangement can be used to monitor and collect health data during a daily routine or sports activity of the person wearing the watch.

According to another aspect of the invention, the light guiding element protrudes from the second surface and away from the first surface. In other words the second surface and the light guiding element are not flush.

The height of the light guiding elements can be chosen such that when placed onto human skin tissue the light guiding element gets in close contact to the surface of the skin. This way, there is no air gap between the light guiding element and the surface of the skin. The losses in light intensity due to refraction can be greatly reduced.

According to another aspect of the invention, the height of protrusion from the second surface is large enough to touch or bend into human skin tissue when the optical sensor arrangement is placed against a human skin.

Typically, a biological probe, and in particular the human skin tissue, have several layers with different consistency and composition. The different layers also have different characteristic depths of penetration which depend on the wavelength of the light emitting device, for example. The coupling of light into and exiting from a tissue can be improved by providing the light emitting element with a certain height of protrusion which relates to the depth of the respective layer.

In the case of an optical heart rate measurement, the light needs to be coupled into the dermis layer. If the light guiding element has a height of protrusion of a couple of millimeters it can bend into the surface of the skin and epidermis layer with an amount large enough to facilitate coupling of light into the dermis layer.

According to another aspect of the invention, the light guiding element is an optical lens. As pointed out above, the light guiding element does not necessarily have to be an optical lens and, thus, have optical properties. However, providing the light guiding element with certain optical properties can have further advantages. For example, the focal length of the optical lens can be adjusted such that most of the light emitted by the light emitting device reaches a certain depth in the biological probe, e.g. once there is a distance between the skin and the optical lens (airgap). In turn, the optical lens can also be arranged to collect a larger amount of light when light is reflected or backscattered from the tissue. In this case, it may also be beneficial to arrange the optical lens so that a large amount of light can be focused or guided onto the light detector.

According to another aspect of the invention, the optical lens comprises a micro lens, a Fresnel lens, a hemispherical lens, a Weierstrass lens or an aspherical lens.

Various optical parameters can be adjusted by the optical design of the light guiding element or the optical lens. For example, a hemispherical lens or a Weierstrass lens provide a large numerical aperture, i.e. the range of angles over which the system can accept or emit light. Furthermore, focal length, aperture and refractive index can also be adjusted to meet cost and ease of production.

According to another aspect of the invention, the light guiding element or elements have a refractive index in the range of a refractive index of human skin tissue. By adapting the refractive index to the human skin tissue losses in light intensity due to refraction can be greatly reduced. Using wavelengths in the visual light the refractive index of human skin tissue lies in the range of 1.33 to 1.55, in the infrared in the range of 1.4 to 1.44 depending on the tissue.

According to another aspect of the invention, the light guiding element comprises a transparent plastic, polymer, or clause. In particular, the light guiding element can be made of polystyrene or polymethylmethacrylate. The choice of material effects the refractive index which may be adapted to the refractive index of human skin tissue. Furthermore, the choice of material also supports making the package skin tolerant, i.e. body fluids such as sweat are prevented from entering the package.

According to another aspect of the invention, the light guiding element is connected to the second surface at a position opposite to the light emitting device.

According to another aspect of the invention, the light guiding element is connected to the second surface at a position opposite to the light detector.

Basically, the light guiding element can be attached or connected to the second surface in different ways an different positions. For example, the light guiding element can be an integral part of the second surface or be fixed to the surface by corresponding structural elements at the surface, e.g. a recess, or base. Alternatively, the light guiding element could also be glued or spray casted to the surface.

According to another aspect of the invention, at least one further light guiding element is connected to the second surface at a position opposite to the light emitting device or opposite to the light detector. For example, one light guiding element is positioned opposite to the light emitting device whereas a second light guiding element is positioned opposite to the light detector. In other words, one light guiding element is used to couple light into a biological tissue whereas the other light guiding element is used to collect and guide light out of the tissue and towards the light detector. Please note, that the aforementioned properties discussed for a single light guiding element apply analogously to all light guiding elements fabricated into the package.

According to another aspect of the invention, the light emitting device is a light emitting diode. In particular, the light emitting diode is an infrared, red, or green light emitting diode. The light emitting device may comprise more than a single light emitting diode.

The depth of penetration of light into a biological probe depends on its wavelength, for example. Typically, infrared light has a higher penetration depth in human skin tissue than the visual light. Furthermore, scattering in the various skin layers also depends on wavelength. For example, the scattering in the dermis varies inversely with wavelength. An infrared wavelength of about 1000 nm has a penetration depth of about 1/1000. In other words, from the point of view of penetration depths it can be beneficial to use infrared light emitting diodes.

According to another aspect of the invention, the optical sensor arrangement comprises a transparent cover, in particular, a glass or plastics cover. The light guiding element or light guiding elements are connected to the transparent cover and are thereby connected to the second surface.

Implementing the second surface is a transparent cover seals the package or optical sensor arrangement from its environment. When the arrangement is used on human skin tissue the cover prevents the light emitting device and light detector as well as electronics from touching the skin directly. The optical sensor arrangement does not require a skin tolerant package design.

According to another aspect of the invention, the package comprises a body of molded material. In particular, the body comprises an opaque molded material. The body of molded material is connected to the first surface of the substrate and comprises the second main surface as well as the optical light barrier.

The body of molded material comprises several cavities. The light emitting device is mounted into the first cavity and the light detector is mounted into a second cavity, respectively. The light emitting device and the light detector mounted into their respective cavities so that they are connected to the first surface. The light guiding element or light guiding elements are connected to the first and second cavity, respectively. The light guiding element light guiding elements can be connected to the cavities and leave part of the cavities open. However, the light guiding elements can also be designed so as to completely seal off the cavities from their environment.

According to another aspect of the invention, the body comprises a third cavity into which a further light emitting device is mounted to the first surface. The light guiding elements are connected to the first, second, and third cavity, respectively. Having more than a single light emitting device implemented in the optical sensor arrangement further improves the signal-to-noise ratio.

According to an aspect of the invention, a smart device watch comprises an optical sensor arrangement according to the principles presented above. In particular, the smart device can be a watch, wrist watch or smart band. In the smart device the optical sensor arrangement is connected into an underside of the smart device.

In the following, the principle presented above will be described in more detail with respect to drawings in which exemplary embodiments are presented. Like components are referenced with like reference numerals.
- Figure 1A: shows an exemplary embodiment of an optical sensor arrangement according to the principle presented,
- Figure 1B: shows another embodiment of an optical sensor arrangement according to the principle presented as seen from above,
- Figure 1C: shows a side view of the embodiment of an optical sensor arrangement according to the principle presented,
- Figure 2: shows another embodiment of an optical sensor arrangement according to the principle presented,
- Figure 3: shows a circuit layout of an embodiment of an optical sensor arrangement according to the principle presented, and
- Figure 4A, and 4B: show an exemplary signal detection scheme in human skin tissue of an embodiment of the optical sensor arrangement according to the principle presented.

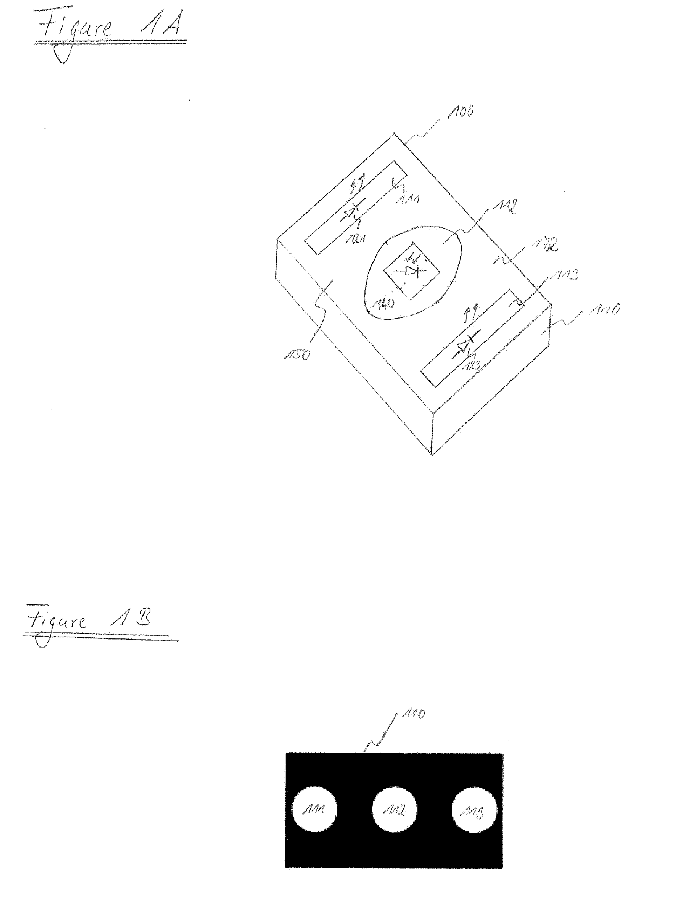

Figure 1A shows an exemplary embodiment of an optical sensor arrangement 100 according to the principle presented. The drawing shows a package 110 for the optical sensor arrangement having a first, second, and third cavity 111, 112, 113. Furthermore, the optical sensor arrangement 100 comprises first and second light emitting devices 121, 123. Preferably, these light emitting devices are light emitting diodes emitting in the near infrared.

The package 110 is made in a molding process and comprises a body of molded material. Preferably, the molded material is opaque with respect to the wavelength of the light emitting devices used in the optical sensor arrangement. In the present embodiment the first and third cavities 111, 113 accommodate the first and second light emitting devices 121, 123, respectively. The first and second light emitting devices 121, 123 are mounted to a substrate 130 which is not depicted in the drawing. However, the first and third cavities 111, 113 are deep enough to accommodate the light emitting devices 121, 123 and allow mounting to the substrate 130. The second cavity 112 has a circular shape and accommodates a light detector 140. Cavity 112 is deep enough to allow mounting of the light detector 140 to the substrate 130.

The section between the light detector 140 and the light emitting devices 121, 123 or, in other words, the room between the first cavity and cavities 111, 113 constitutes an optical light barrier 150. The optical light barrier 150 prevents or blocks light emitted from the light emitting devices 121, 123 from directly reaching the light detector 140. Typically, the molded material of the body and also the material of the substrate 130 absorb or at least attenuate light emitted from the light emitting devices 121, 123 strong enough to render optical crosstalk between the light detector 140 and light emitting devices 121, 123 negligible.

Figure 1B shows an embodiment of an optical sensor arrangement according to the principle presented as seen from above. The drawing shows the package 110 having the molded body and the first, second, and third cavity 111, 112, 113 designed into the mold material. In this case the cavities all have circular shape. Similar to the embodiment of Figure 1A the cavities 111, 112, 113 can also have different shape, like a rectangular shape.

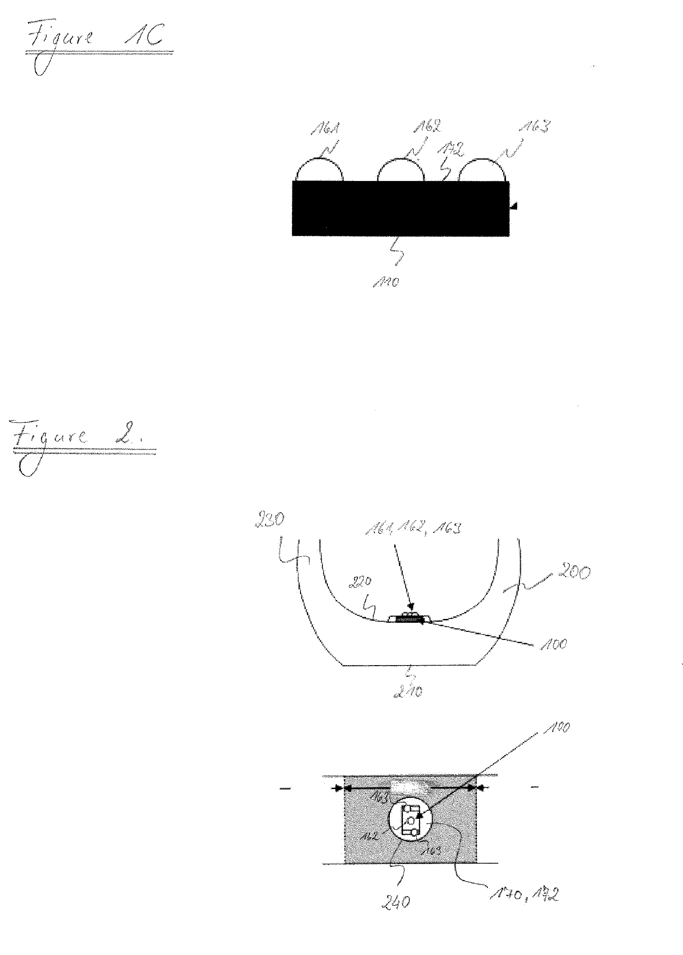

Figure 1C shows a side view of an embodiment of an optical sensor arrangement according to the principle presented. In this drawing, the package 110 and the body of molded material are provided with several light guiding elements. In this particular design, there is a first, second, and third light guiding element 161, 162, 163 which are connected to the first, second, and third cavity 111, 112, 113, respectively via a second surface 172.

According to this embodiment, the light guiding elements 161, 162, 163 are implemented as optical micro lenses having a spherical surface and planar surface. Typically, the lenses have a diameter of about 1 to 5 mm, for example 2 mm. Not shown in the drawing, however, are the respective positions on the light detector 140 and the first and second light emitting devices 121, 123. Preferably, these devices are aligned with the optical axis of the first, second, and third optical lenses 161, 162, 163, respectively.

Figure 2 shows another embodiment of an optical sensor arrangement according to the principle presented. The drawing shows a possible way to implement an exemplary optical sensor arrangement 100 into a wrist watch 200.

The upper part of the drawing shows the wrist watch 200 having the optical sensor arrangement 100 in a side view. The watch 100 comprises a display housing 210 comprising the watch electronics and an underside 220 as well as a strap 230 to attach the watch around the wrist of the person. The optical sensor arrangement 100 is attached to the underside 220 of the watch.

The optical sensor arrangement 100 in this embodiment comprises three light guiding elements, the first, second, and third optical lenses 161, 162, 163. The optical lenses are connected to a glass cover 170 which covers the package 110 made from the body of molded material. In this case, the glass cover 170 comprises the second surface 172 to which the optical lenses 161, 162, 163 are attached to. The glass cover 170 also seals off the optical sensor arrangement 100 from environmental influences like sweat. The optical lenses 161, 162, 163 protrude from the second surface 172, i.e. from the glass cover 170. The particular height of the lenses is sufficient to touch or bend into a human skin tissue as soon as the watch is attached to the wrist of a person. Typically, the height of the lenses is in the range of a couple of millimeters. The optical lenses 161, 162, 163 therefore serve two purposes. First, they keep the optical sensor arrangement 100 mechanically fixed to the wrist. Second, because of their height they allow to push the optical sensor arrangement 100 into the dermis layer of the skin in order to improve quality of detection of light detector 140.

The bottom part of the drawing shows the underside 220 of the wrist watch 200. Strap 230 is connected to the left and right side of the wrist watch 200. The underside 220 of the wrist watch 200 has a circular opening 240. The optical sensor arrangement 100 is mounted into the watch 200 via this opening 240. In addition, the glass cover 170 is fitted into the circular opening 240 and may somewhat protrude from the underside 220 of the watch 200. The optical lenses 161, 162, 163 are connected to the glass cover 170 and further protrude from the second surface 172 of the cover 170 (see also Figure 1A).

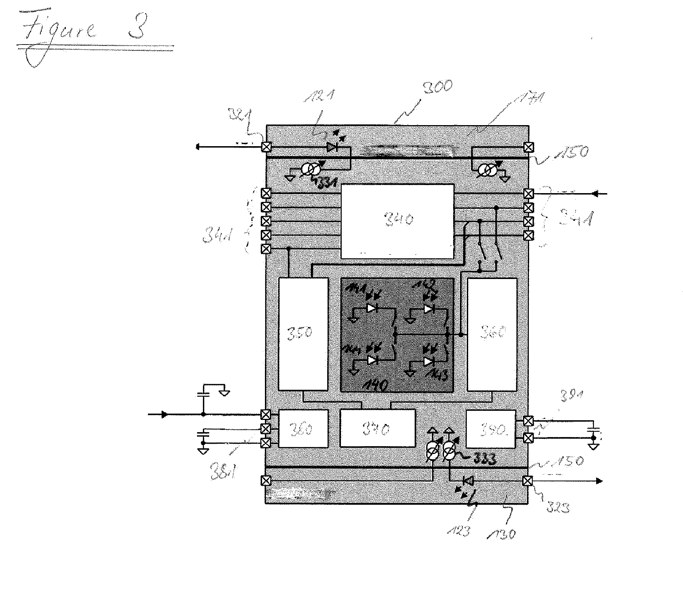

Figure 3 shows an exemplary circuit layout 300 of an embodiment of an optical sensor arrangement 100 according to the principle presented. The drawing depicts the substrate 130 without the package 100 mounted on top of a first surface 171 of the substrate. Only the optical light barrier 150 is indicated in the drawing.

The light emitting devices 121, 123 are connected to supply terminals 321, 323 and current sources 331, 333, respectively. The circuit layout 300 also comprises a microprocessor 340, an electrical front end 350, an optical front end 360 and analog-to-digital converter 370. Furthermore, the layout provides a low dropout regulator 380 and biasing means 390.

The microprocessor 340 is used to coordinate the optical measurement, i.e. of biological parameters like the heart rate. The microprocessor 340 has connections to general-purpose input/output terminals 341 in order to provide a sensor signal.

The optical front end 360 is connected to the light detector 140. The light detector 140 comprises four individual photodiodes 141, 142, 143, 144 which can be switched by means of the optical front end 360. This way, a single photodiode, two, three or all for photodiodes can be activated at a time. The optical front end 360 is also used to collect sensor signals from the photodiodes 141, 142, 143, 144 and transmit the signals to the analog to digital converter 370. The low dropout regulator 380 provides a supply voltage to the circuit layout 300 and the biasing means 390 are used to prebias the circuit. The low dropout regulator 380 and biasing means 390 are connected to respective terminals 381, 391.

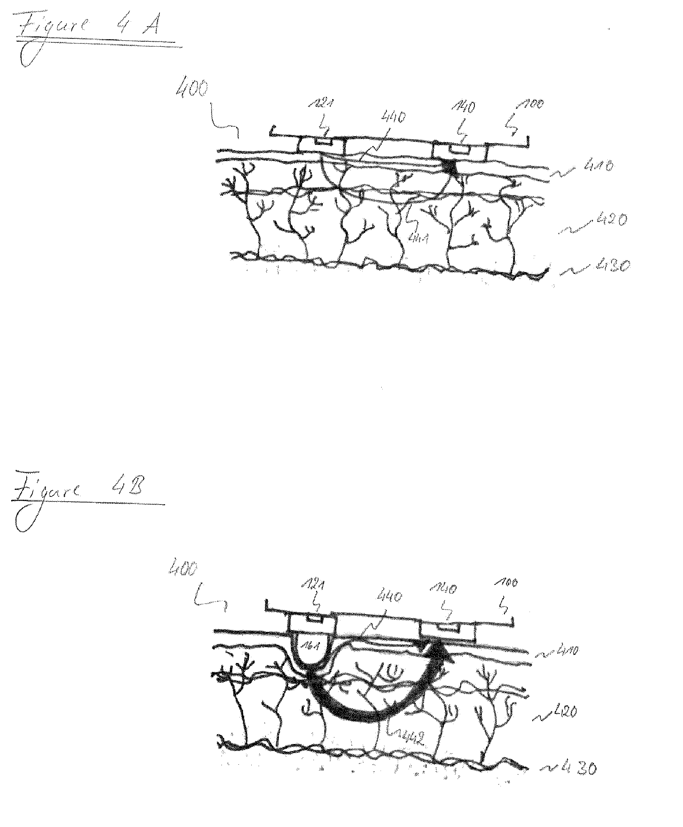

The details of operation are common to all aforementioned embodiments of the optical sensor arrangement and will be discussed in further detail with respect to Figure 4A and 4B. Figures 4A, and 4B show an exemplary signal heart rate detection scheme in human skin tissue of an embodiment of an optical sensor arrangement according to the principle presented.

In order to provide an optical measurement of biological parameters, e.g. of a person's heart rate the optical sensor arrangement 100 must be placed in close vicinity to the human skin tissue 400. Preferably, the optical sensor arrangement 100 is mounted into the wrist watch 200 and close to the wrist of a person. The latter situation is depicted in Figure 4A and 4B.

Figure 4A shows an embodiment of an optical sensor arrangement 100 without having light guiding elements such as the optical lenses introduced above. The human skin is comprised by several skin layers 410, 420, 430. Closest to the optical sensor arrangement 100 is the stratum corneum (not shown) and the epidermis 410, followed by the dermis 420 and hypodermis 430. Especially for a heart rate measurement based on PPG principles, the dermis layer 420 needs to be addressed by the optical sensor arrangement 100. In fact, the epidermis 410 does not have any blood vessels and therefore carries no pulsatile blood. The stratum corneum is about 10 µm thick, the epidermis 410 about 100 µm, and the dermis 420 has a mean thickness of about 3 mm.

The optical sensor arrangement 100 can be used to perform a PPG-based optical heart rate measurement. This method works according to the following principles. One or more light emitting devices 121 are used to couple light into the skin 400 which enters into the several skin layers 410, 420, 430. Generally speaking the light can be reflected, scattered or absorbed in the different skin layers. For example, depending on the depth of penetration, light reaches the dermis layer 420. The tissue is able to reflect and scatter light so that eventually the light travels along the layer (see arrows 440, 441). Some amount of the light gets back scattered and may leave the skin layers at a position close to the light detector 140. This portion of light can be detected so the light detector 140 generates a corresponding sensor signal. The blood pressure of the person wearing the wrist watch 200 with the optical sensor arrangement 100 changes during the measurement. The change in blood pressure also changes the diameter of the blood vessels and light traveling in or along these blood vessels is slightly modulated by this physical movement. As a consequence the sensor signal includes a modulation. This modulation can be demodulated inside the watch electronics or, alternatively in the microprocessor included in the circuit layout 300. The demodulated sensor signal is a measure of the heart rate.

The optical paths of light traveling through the different skin layers is affected by several the scattering and reflection events and other distortions. Only a rather small amount of light intensity is able to reach the light detector (see arrows 440, 441). An optical sensor arrangement as the one used in Figure 4A therefore only has a small signal-to-noise ratio. The light guiding elements 161, 162, 163 introduced above improve the signal quality which can be achieved in a measurement of biological parameters and increase the signal-to-noise ratio. This improvement is depicted in Figure 4B (see arrow 442).

Figure 4B shows an embodiment of an optical sensor arrangement 100 according to the principle presented. In this case, only a single light guiding element 161 or optical lenses present and mounted in front of the light emitting device 121. The optical lens 161 has a height of protrusion which is sufficient to bend into the human skin and thus is in close contact to the human skin tissue 400. The bold arrow 442 in the drawing indicates the increased coupling of light into the skin tissue 400. Because of the optical lens 161 and its height of protrusion most of the light is coupled into the epidermis layer 420.

The apparent increase of intensity of light traveling through the skin layer can also be measured under defined conditions. These results are summarized in table 1. The results have been collected using different measurement setups. The reference setup corresponds to the optical sensor arrangement discussed in Figure 4A. Three further measurement setups have been used to show the impact of the light guiding elements. The setups basically corresponds to the one shown in Figure 1C, i.e. three light guiding elements 161, 162, 163 are present. In one setup the light guiding elements or optical lenses 161, 162, 163 have been centred with respect to the light detector 140 and the light emitting device 121, 123. In the other setups the optical lenses 161, 162, 163 have been offset by 1 mm and 2 mm, respectively.

In order to highlight the effect of the optical lens the AC amplitude has been recorded. The supply currents of the light emitting device, in this case the light emitting diode 121, 123 have been adjusted to a similar level.

The results show an increase of AC amplitude by about one order of magnitude. The setup having the centred optical lens shows the highest increase. However, also small offsets of the optical axis of the lenses show a similar but slightly smaller increase. This indicates that the optical sensor arrangement equipped with the light guiding elements is rather robust to small offsets. In fact, similar results have been achieved with light guiding elements that do not have an ideal optical lens shape. This reduces the requirements on the optical design of the light guiding elements and further helps reducing cost of the optical sensor arrangement.

**Table 1**

| **measurement setup** | **AC amplitude / ADC counts peak-to-peak** | **LED current / mA** | **AC amplitude / mA** |
|---|---|---|---|
| **reference** | 1700 | 1,3 | 13080 |
| **centred** | 32000 | 1,0 | 32000 |
| **1mm from center** | 24000 | 1,2 | 20000 |
| **2mm from center** | 28000 | 1,6 | 17500 |

### Reference numerals

- 100: optical sensor arrangement
- 110: package
- 111: first cavity
- 112: second cavity
- 113: third cavity
- 121: light emitting device
- 123: light emitting device
- 130: substrate
- 140: light detector
- 141: photodiode
- 142: photodiode
- 143: photodiode
- 144: photodiode
- 150: optical light barrier
- 161: light guiding element
- 162: light guiding element
- 163: light guiding element
- 170: glass cover
- 171: first surface
- 172: second surface
- 200: watch
- 210: display housing
- 220: underside
- 230: strap
- 240: opening
- 300: circuit layout
- 321: supply terminal
- 323: supply terminal
- 331: current source
- 333: current source
- 340: micro processor
- 341: general-purpose input/output terminal
- 350: electronic front end
- 360: optical front end
- 370: analog to digital converter
- 380: low dropout regulator
- 381: terminal
- 390: biasing means
- 391: terminal
- 400: human skin tissue
- 410: epidermis
- 420: dermis
- 430: hypo dermis
- 440: arrow
- 441: arrow
- 442: arrow

## Claims

1. Optical sensor arrangement for an optical measurement of biological parameters, in particular for an optical heart rate measurement, comprising:
- a light emitting device (121, 123) and a light detector (140),
- a substrate (130) comprising a first main surface (171) onto which the light emitting device (121, 123) and the light detector (140) are mounted,
- a package (110) enclosing the light emitting device (121, 123), the light detector (140) and the substrate (130), comprising a second main surface (172) facing the first main surface (171), and further comprising an optical light barrier (150) designed to block light to be emitted by the light emitting device (121, 123) from directly reaching the light detector (140), and
- a light guiding element (161, 162, 163) connected to the second main surface (172) and designed for coupling light to be emitted by the light emitting device (121, 123) into and/or out of a human skin tissue (400).

2. Optical sensor arrangement according to claim 1 wherein the light guiding element (161, 162, 163) protrudes from the second surface (172).

3. Optical sensor arrangement according to claim 2 wherein a height of protrusion from the second surface (172) is large enough to touch or bend into human skin tissue (400) when the optical sensor arrangement is placed against a human skin.

4. Optical sensor arrangement according to one of claims 1 to 3, wherein the light guiding element (161, 162, 163) is an optical lens.

5. Optical sensor arrangement according to claims 4, wherein the optical lens is a micro lens, a Fresnel lens, a hemispherical lens, Weierstrass lens or an aspherical lens.

6. Optical sensor arrangement according to one of claims 1 to 5, wherein the light guiding element (161, 162, 163) has a refractive index in the range of a refractive index of human skin tissue.

7. Optical sensor arrangement according to one of claims 1 to 6, wherein the light guiding element (161, 162, 163) comprises a transparent plastic, polymer or glass, in particular, polystyrene, polymethylmethacrylate.

8. Optical sensor arrangement according to one of claims 1 to 7, wherein the light guiding element (161, 162, 163) is connected to the second surface (172) at a position opposite to the light emitting device (121, 123).

9. Optical sensor arrangement according to one of claims 1 to 7, wherein the light guiding element (161, 162, 163) is connected to the second surface (172) at a position opposite to the light detector (140).

10. Optical sensor arrangement according to one of claims 1 to 9, comprising at least one further light guiding element (161, 162, 163) connected to the second surface (172) at a position opposite to the light emitting device (121, 123) or the light detector (140), respectively.

11. Optical sensor arrangement according to one of claims 1 to 10 wherein the light emitting device (161, 162, 163) is a light emitting diode, in particular an infrared light emitting diode.

12. Optical sensor arrangement according to one of claims 1 to 11, comprising a transparent cover (170), in particular a glass or plastics cover, connected to the second surface (172) wherein the light guiding element (161, 162, 163) are connected to the second surface (172) by means of the transparent cover (170).

13. Optical sensor arrangement according to one of claims 1 to 12 wherein the package (110) comprises
- a body of molded material, in particular a body of opaque molded material, connected to the first surface (171) and comprising the second main surface (172) and the optical light barrier (150),
- a first cavity (111) in the body into which the light emitting device (121) is mounted to the first surface (171), and
- a second cavity (112) in the body into which the light detector (140) is mounted to the first surface (171), and
- the light guiding element and the further light guiding element (161, 162) connected to the first or second cavity (111, 112), respectively.

14. Optical sensor arrangement according to claim 13, wherein
- the body comprises a third cavity (113) into which into which a further light emitting device (123) is mounted to the first surface (171), and
- the light guiding elements (161, 162, 163) are connected to the first, second, and third cavity (111, 112, 113), respectively.

15. Smart device, in particular a watch, a wrist watch or a smart band; comprising an optical sensor arrangement according to one of claims 1 to 14, wherein the optical sensor arrangement is connected into an underside (220) of the watch.
